# FASCICULE DE BREVET EUROPEEN

(11) **EP 3 016 745 B1**
(45) Date de publication et mention de la délivrance du brevet: **30.08.2017**
(21) Numéro de dépôt: 14755148.5
(22) Date de dépôt: 03.07.2014
(51) Int. Cl.: B03B 9/06, B07B 13/11, B07B 1/34, B07B 1/52, C05F 9/02, B07B 1/46

(54) **PROCEDE ET INSTALLATION DE TRAITEMENT DE DECHETS AVEC CRIBLAGE A OSCILLATIONS**
ABFALLVERARBEITUNGSVERFAHREN UND -ANLAGE MIT SCHWINGENDEN SIEBEN
A WASTE TREATMENT PROCESS AND PLANT WITH OSCILLATING SCREENING

(30) Priorité: 05.07.2013 FR 1356624
(43) Date de publication de la demande: 11.05.2016
(73) Titulaire: SUEZ Groupe, 92040 Paris la Défense Cedex (FR)
(72) Inventeur: SOMMAIN, Arnaud, F-34110 Mireval (FR)
(74) Mandataire: Cabinet Armengaud Aîné
(86) Numéro de dépôt international: PCT/IB2014/062832
(87) Numéro de publication internationale: WO 2015/001514

(56) Documents cités:
- EP-A2- 2 364 782
- FR-A1- 2 577 448
- GB-A- 1 204 551
- US-A- 4 350 591

## Description

La présente invention est relative à un procédé de traitement de déchets, en particulier d'ordures ménagères, contenant des matières organiques mélangées à des indésirables, notamment métaux, matières minérales, plastique, verre, procédé selon lequel :
- les déchets sont soumis à un premier tri par criblage,
- la fraction des déchets traversant le criblage est soumise à un traitement de pré-fermentation dans un tube rotatif avec alimentation à une extrémité et extraction à l'autre extrémité,
- au moins une partie de la matière sortant du tube de traitement de pré-fermentation est soumise à un traitement de méthanisation dans un digesteur.

Le document EP 2 364 782 A2 décrit un procédé de traitement de déchets selon le préambule de la revendication 1, et un dispositif de traitement de déchets selon le préambule de la revendication 2. Le document FR2577448A1 décrit un moyen de criblage pour matière pré-fermentée, dans lequel le criblage s'effectue sur un tamis comprenant une grille située dans un plan incliné d'un angle a par rapport à l'horizontale, la grille étant soumise à des vibrations selon une direction sensiblement orthogonale à la direction d'écoulement des déchets au dessus du tamis incliné. Les déchets ménagers issus de collectes non sélectives contiennent différentes catégories de matériaux, telles que déchets putrescibles (déchets alimentaires, déchets verre), papier, carton, verre, plastique, métaux ferreux ou non ferreux, tissus, textiles sanitaires, produits toxiques (piles électriques, pots de peinture).

La méthanisation et le compostage de ces déchets ménagers en vue de les transformer en biogaz et en compost valorisable comporte généralement quatre étapes principales :
- une préparation mécanique des déchets qui vise à séparer les matières organiques biodégradables des autres fractions non valorisables en biogaz ou en compost ; un tube rotatif de pré-fermentation, généralement essentiellement horizontal, constitue un moyen pour une telle préparation;
- la méthanisation qui vise à produire une énergie renouvelable et est réalisée dans des enceintes horizontales ou verticales, agitées mécaniquement ou non ;
- le compostage, ou maturation aérobie, qui fait généralement intervenir une opération préalable de pressage du digestat provenant de la méthanisation, afin d'atteindre un niveau de teneur en matières sèches et une porosité permettant l'autocompostage du digestat, ou alors nécessite l'apport d'un agent structurant et son mélange avec le digestat pour obtenir un substrat compostable ;
- l'affinage final qui vise à retirer de façon complémentaire les contaminants restant après les deux opérations précédentes, et à préparer le compost à une granulométrie permettant sa valorisation agronomique.

Le traitement de ces déchets nécessite donc un tri qui permettra notamment de séparer les matières organiques biodégradables des autres fractions non valorisables en biogaz ou en compost.

Le domaine technique particulier de l'invention s'inscrit donc dans le contexte particulier de cette première étape de préparation mécanique des déchets.

Le criblage permet de sélectionner les matériaux suivant leur granulométrie et de séparer matières organiques et matières minérales.

Un criblage fin utilisant une maille fine permet d'optimiser la collecte des matières organiques qui pourront être valorisées.

Malheureusement, à l'heure actuelle, il est difficile d'utiliser une maille fine sans rencontrer de problèmes de colmatage. Les dispositifs existants utilisent donc des mailles de taille relativement importante évitant la perte de matière organique mais n'offrant pas la sélectivité nécessaire à la purification de celle-ci.

L'invention a donc pour but, surtout, de proposer un procédé de traitement des déchets tel que présenté en préambule, et comportant notamment un criblage utilisant des mailles fines tout en minimisant les problèmes de colmatage.

Selon l'invention un procédé de traitement des déchets, en particulier d'ordures ménagères, contenant des matières organiques mélangées à des indésirables, notamment métaux, matières minérales, plastiques, verre, procédé selon lequel :
- les déchets sont soumis à un premier tri par criblage,
- la fraction des déchets traversant le criblage est soumise à un traitement de pré-fermentation dans un tube rotatif avec alimentation à une extrémité et extraction à l'autre extrémité,
- au moins une partie de la matière sortant du tube de traitement de pré-fermentation est soumise à un traitement de méthanisation dans un digesteur,
est caractérisé en ce que les déchets, après traitement de pré-fermentation, sont soumis à un tri par criblage sur un tamis comprenant une grille située dans un plan incliné d'un angle α par rapport à l'horizontale, la grille étant soumise à des vibrations selon une direction transversale sensiblement orthogonale à la direction d'écoulement des déchets au dessus du tamis incliné.

L'invention est également relative à une installation pour la mise en oeuvre du procédé, cette installation comprenant :
- un premier crible primaire,
- un tube rotatif de pré-fermentation,
- et un digesteur pour une fraction fine issue du tube de pré-fermentation,
   la grille pouvant comprendre une série de fils, la ligne moyenne de chaque fil présentant une orientation proche de la direction d'écoulement des déchets au-dessus du tamis, les fils étant situés sur un même plan, la distance entre un fil et ses fils voisins variant entre un maximum et un minimum, la distance minimum étant non-nulle. Les fils peuvent présenter une forme sensiblement sinusoïdale.

Avantageusement, la grille comprend au moins une barre transversale, disposée perpendiculairement à la ligne moyenne des fils et solidaire des fils de manière à maintenir un écartement constant entre les fils. Les vibrations peuvent être transmises au tamis par l'intermédiaire de la barre transversale. Un marteau électromagnétique peut frapper une extrémité de la barre transversale.

Avantageusement la maille L de la grille est inférieure ou égale à 5 mm. La maille L de la grille peut également être inférieure ou égale à 2 mm.

L'angle α peut être compris entre 30° et 60°. De préférence l'angle α est de 42°.

Un moyen de brossage peut être apte à exercer un mouvement de va-et-vient sur le tamis.

Le moyen de brossage peut comprendre au moins un rouleau d'axe sensiblement orthogonal à la direction d'écoulement des déchets. Le rouleau peut être monté sur un châssis mobile.

Le moyen de brossage peut comprendre un bras articulé équipé d'une brosse.

D'autres caractéristiques et avantages de l'invention apparaîtront dans la description qui suit d'un mode de réalisation avec référence aux dessins annexés mais qui n'a aucun caractère limitatif. Sur ces dessins :
Fig. 1 est un diagramme représentant le fonctionnement du procédé selon l'invention,
Fig. 2 est une vue schématique en perspective d'un tamis utilisé dans le procédé selon l'invention,
Fig. 3 est une vue schématique de dessus de la grille du tamis de Fig. 2,
Fig. 4 est une vue schématique à plus grande échelle d'un détail de Fig. 3,
Fig. 5 est une vue schématique en perspective d'un tamis utilisé dans le procédé selon l'invention, équipé d'un dispositif de brossage à rouleaux, Fig. 6 est une vue en élévation latérale du tamis de Fig. 5,
Fig. 7 est une vue similaire à Fig. 6, un support escamotable étant déployé,
Fig. 8 et Fig. 9 sont des vue similaires à Fig. 7, le support escamotable se déplaçant pour effectuer le nettoyage,
Fig. 10 est une vue similaire à Fig. 6, en fin de nettoyage,
Fig. 11 et Fig. 12 sont des vues similaires à Fig. 5 illustrant la position du chariot au-dessus d'une grille et d'une autre voisine, et
Fig. 13 est une vue similaire à Fig. 11 et 12, le chariot étant en position d'attente,

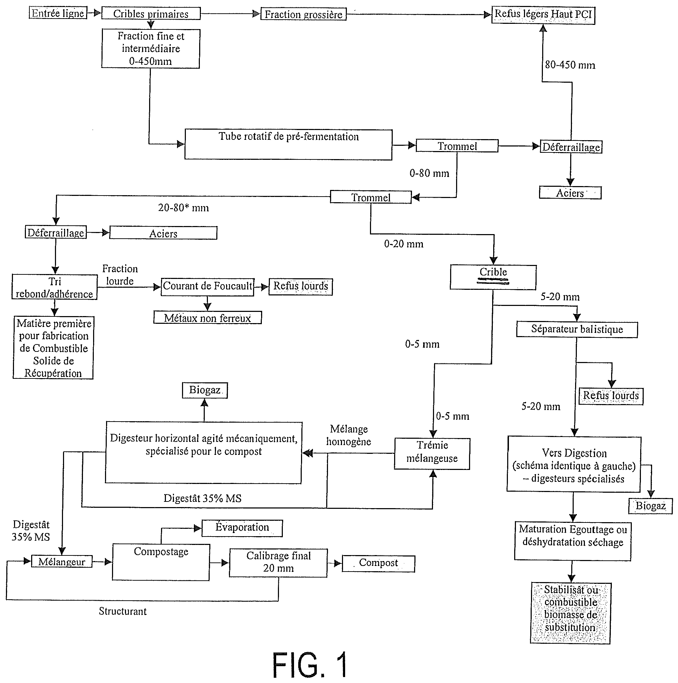

Fig. 1 permet de replacer l'invention dans son contexte. On peut voir en haut à gauche l'entrée ligne où les déchets à traiter sont introduits. Après un premier crible dit primaire, la fraction fine de granulométrie inférieure à 450 mm passe par un tube rotatif de pré-fermentation puis par un trommel où la fraction la plus fine, de granulométrie inférieure à 80 mm, est récupérée et passe par un second trommel où la fraction la plus fine, de granulométrie inférieure à 20 mm, est récupérée pour être passée sur un crible, c'est-à-dire le tamis selon l'invention, qui permet de séparer une première fraction très fine, de granulométrie inférieure à 5 mm d'une seconde fraction de granulométrie comprise entre 5 et 20 mm.

Par la suite la première fraction passe par un digesteur horizontal agité mécaniquement avec une recirculation au moins partielle par l'intermédiaire d'une trémie mélangeuse.

C'est lors de cette phase de digestion que du biogaz est extrait. Le digestat est lui composté.

La seconde fraction passe d'abord par un séparateur balistique permettant d'écarter des déchets lourds avant de passer par un digesteur de façon similaire à la première fraction. Le digestat subit une phase de maturation par égouttage ou déshydratation pour le stabiliser ou le transformer en combustible.

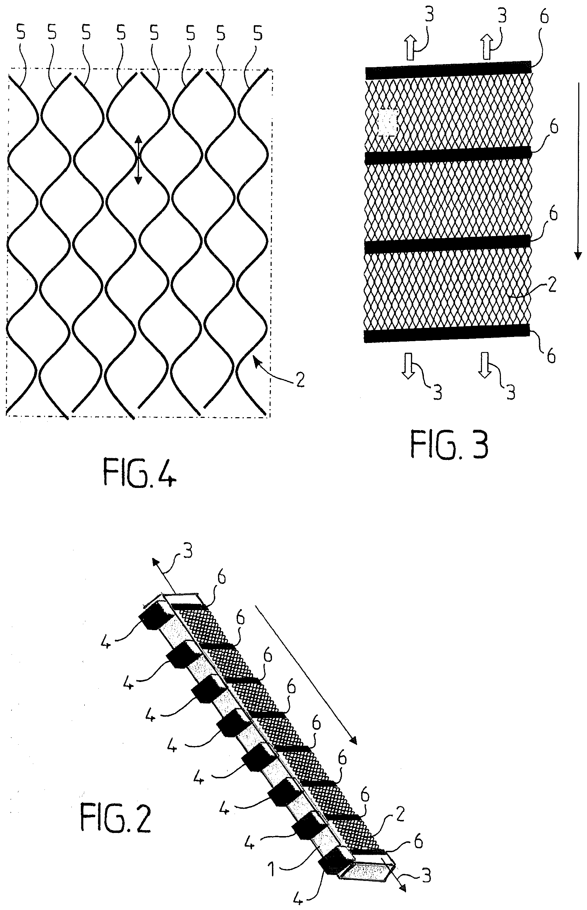

Fig. 2, on peut voir le dispositif de criblage utilisé dans le procédé selon l'invention.

Le dispositif de criblage comprend un châssis 1 maintenant une grille 2 inclinée. La grille 2 est située dans un plan incliné d'un angle α sur l'horizontale.

Avantageusement, la grille 2 est montée sur un support de grille statique d'inclinaison réglable de 30 à 60 degrés. Dans ce mode de réalisation l'inclinaison est de 42° par rapport à l'horizontale.

Cette inclinaison importante permet au produit à traiter de dévaler la pente sans être forcé de passer à travers une maille. Le colmatage est ainsi beaucoup moins fréquent.

La grille 2 présente une forme allongée, une extrémité inférieure et une extrémité supérieure étant solidaires de systèmes de tension 3 permettant d'imprimer une tension permanente à la grille 2 pour la maintenir sensiblement plane.

Des marteaux électromagnétiques 4 permettant de frapper latéralement la grille sont montés sur les côtés de la grille solidairement du châssis 1.

La grille 2 (Fig. 4) se présente sous la forme d'une série de fils longitudinaux 5 présentant un profil sensiblement sinusoïdal organisés selon une forme rectangulaire.

Les fils 5 se situent sur un même plan, la distance entre un fil 5, et ses fils voisins variant entre un maximum et un minimum. Mais, même lorsque la distance entre un fil et son fil mitoyen est minimum, aucune soudure n'est présente entre les deux fils. Cette disposition particulière a été retenue après de nombreux essais. Elle minimise le colmatage et facilite le nettoyage de la grille.

Des barres transversales 6 de raidissage sont prévues à intervalles réguliers pour maintenir un écartement constant entre les fils 5.

Les marteaux électromagnétiques 4 sont disposés en vis-à-vis des barres de raidissage. Les fréquences utilisées sont généralement comprises entre 10 et 60Hz. Dans le cas de l'utilisation de plusieurs étages de criblage, il est possible d'utiliser des fréquences différentes pour chaque étage.

Pour garantir le fonctionnement du procédé, une étape de nettoyage est prévue.

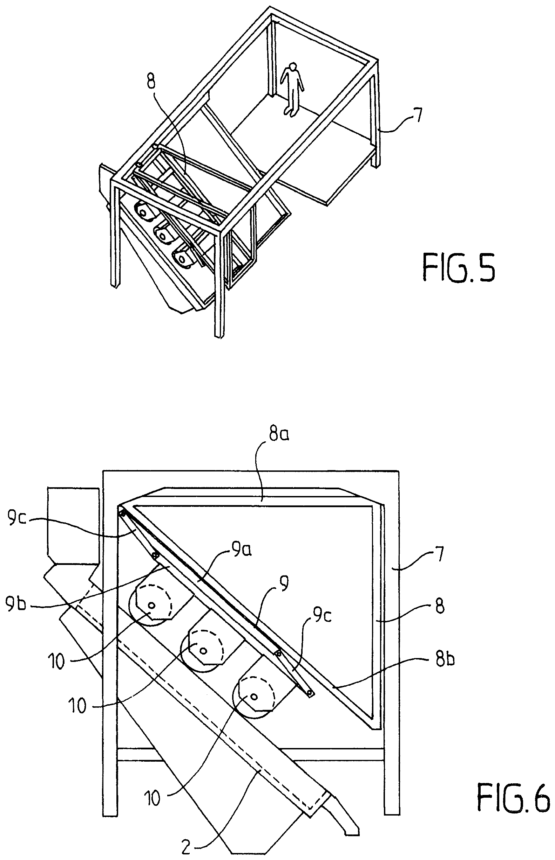

Fig. 5 on peut voir une charpente métallique 7 placée au dessus de la grille 2. Un chariot 8 est suspendu sous la charpente 7 par l'intermédiaire de rails de manière à pouvoir glisser transversalement par rapport à la grille 2. Le chariot 8 est constitué de profilés métalliques assemblés entre eux pour délimiter notamment une surface supérieure 8a, sur laquelle sont fixés des galets coopérants avec les rails prévus sur la charpente 7, et une surface inférieure inclinée 8b, parallèle à la surface de la grille 2, sous laquelle est montée un support escamotable 9 supportant des rouleaux de nettoyage 10. Des moyens non représentés sont prévus pour l'entrainement en rotation des rouleaux autour de leur axe.

Le support escamotable 9 comporte deux faces parallèles 9a et 9b reliées par des biellettes 9c. La première face 9a est montée coulissante sous le chariot 8 tandis que la seconde face 9b supporte les rouleaux de nettoyage 10.

Il est prévu deux grilles 2 (d'un mètre 75 sur trois mètres 60), disposées côte à côte et alimentées par une goulotte en V non représentée. Cette goulotte permet d'alimenter les deux grilles 2 simultanément ou bien simplement l'une d'entre elles.

De cette façon il devient possible de diminuer temporairement le débit de déchets arrivant sur le poste de criblage tout en orientant la totalité du flux sur l'une des deux grilles pour permettre le nettoyage de l'autre grille en flux tendu mais sans la présence de déchets.

Le chariot 8 peut donc se trouver au dessus de l'une ou l'autre des grilles ou bien déporté sur le côté, en position d'attente.

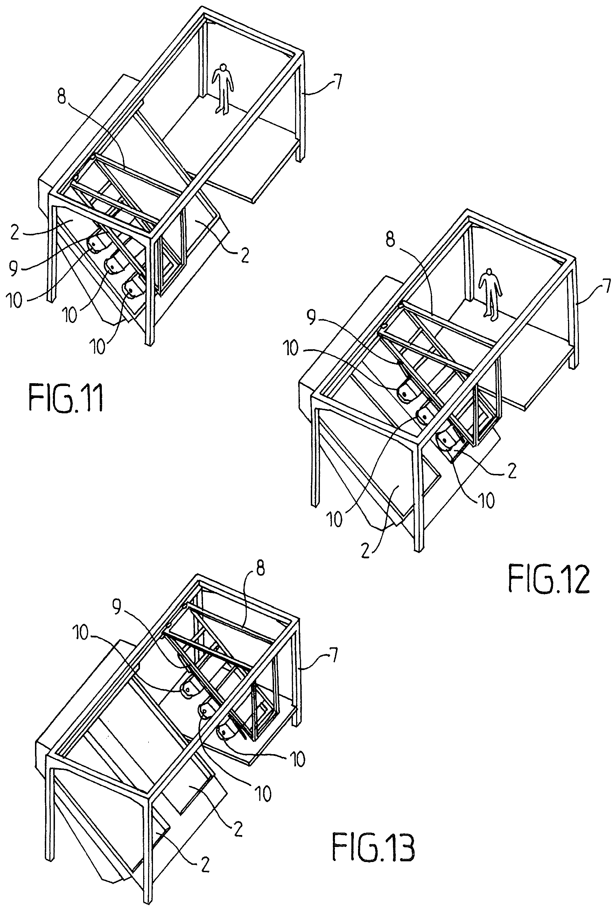

En fonctionnement, le chariot 8 est disposé en position d'attente comme sur Fig. 13.

Lorsqu'une opération de nettoyage est estimée nécessaire, le chariot 8 est déplacé au-dessus de l'une des grilles 2 dont le nettoyage va être effectué (Fig. 11 et 12). Le support escamotable 9 se trouve alors en position repliée comme illustré Fig. 6.

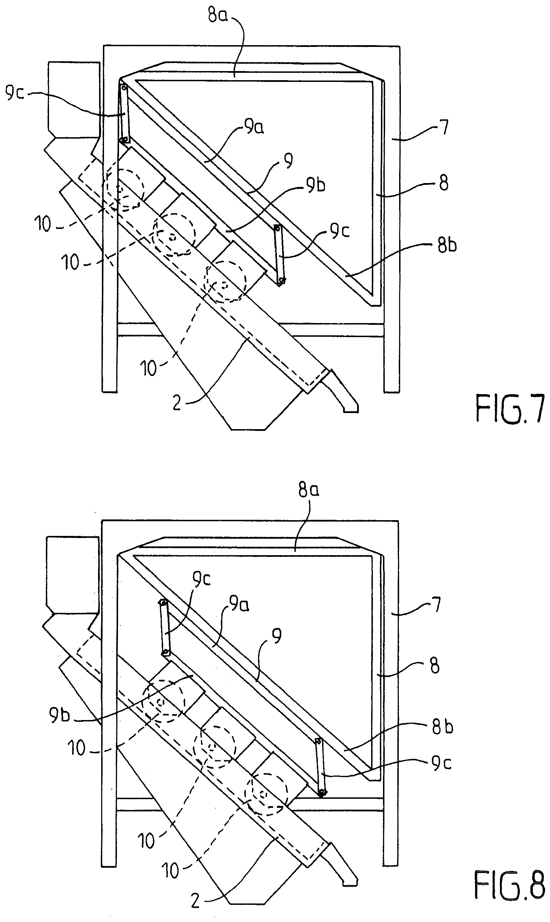

Une fois le chariot 8 arrivé en position au dessus de la grille 2 à nettoyer, le support escamotable 9 est déplié (Fig. 7) pour mettre les rouleaux 10 en contact avec la grille 2.

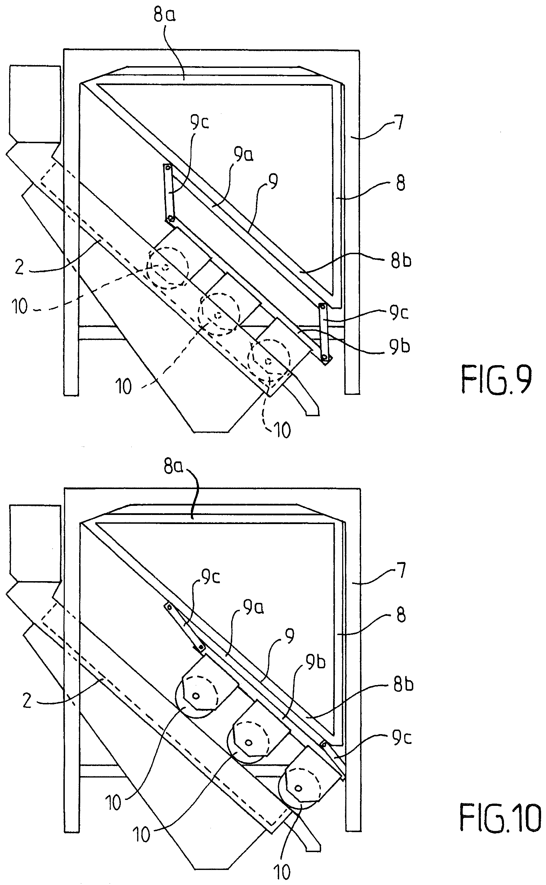

Le support escamotable 9 effectue alors une translation vers le bas par rapport au chariot 8, parallèlement à la grille 2 pour en effectuer le nettoyage (Fig. 8 et 9).

Une fois le nettoyage de la grille 2 effectué le support escamotable 9 est replié (Fig. 10) avant le retour du chariot 8 en position d'attente (Fig. 13).

## Revendications

1. Procédé de traitement des déchets, en particulier d'ordures ménagères, contenant des matières organiques mélangées à des indésirables, notamment métaux, matières minérales, plastiques, verre, procédé selon lequel :
- les déchets sont soumis à un premier tri par criblage,
- la fraction des déchets traversant le criblage est soumise à un traitement de pré-fermentation dans un tube rotatif avec alimentation à une extrémité et extraction à l'autre extrémité,
- au moins une partie de la matière sortant du tube de traitement de pré-fermentation est soumise à un traitement de méthanisation dans un digesteur,
- les déchets, après traitement de pré-fermentation, sont soumis à un tri par criblage,
**caractérisé en ce que** le tri par criblage après traitement de pré-fermentation est réalisé sur un tamis comprenant une grille (2) située dans un plan incliné d'un angle α par rapport à l'horizontale, la grille (2) étant soumise à des vibrations selon une direction transversale sensiblement orthogonale à la direction d'écoulement des déchets au-dessus du tamis incliné.

2. Installation pour la mise en oeuvre du procédé selon la revendication 1, comprenant :
- un premier crible primaire,
- un tube rotatif de pré-fermentation,
- un digesteur pour une fraction fine issue du tube de pré-fermentation,
- un crible positionné après le tube rotatif de pré-fermentation, **caractérisée en ce que** le crible est un tamis comprenant une grille (2) située dans un plan incliné d'un angle α par rapport à l'horizontale, la grille (2) étant soumise à des vibrations selon une direction transversale sensiblement orthogonale à la direction d'écoulement des déchets au-dessus du tamis incliné,
et **en ce que** la grille (2) comprend une série de fils (5), la ligne moyenne de chaque fil (5) présentant une orientation proche de la direction d'écoulement des déchets au-dessus du tamis, les fils (5) étant situés sur un même plan, la distance entre un fil (5) et ses fils voisins variant entre un maximum et un minimum, la distance minimum étant non-nulle.

3. Installation de traitement des déchets selon la revendication 2, **caractérisée en ce que** les fils (5) présentent une forme sensiblement sinusoïdale.

4. Installation de traitement des déchets selon la revendication 2 ou 3, **caractérisée en ce que** la grille (2) comprend au moins une barre transversale (6), disposée perpendiculairement aux fils (5) et solidaire des fils (5) de manière à maintenir un écartement constant entre les fils (5).

5. Installation de traitement des déchets selon la revendication 4, **caractérisée en ce que** les vibrations sont transmises au tamis par l'intermédiaire de la barre transversale (6).

6. Installation de traitement des déchets selon la revendication 5, **caractérisée en ce qu'**un marteau électromagnétique (4) frappe une extrémité de la barre transversale (6).

7. Installation de traitement des déchets selon l'une quelconque des revendications 2 à 6, **caractérisée en ce que** la maille L de la grille (2) est inférieure ou égale à 5 mm.

8. Installation de traitement des déchets selon la revendication 7, **caractérisée en ce que** la maille L de la grille (2) est inférieure ou égale à 2 mm.

9. Installation de traitement des déchets selon l'une quelconque des revendications 2 à 8, **caractérisée en ce que** l'angle α est compris entre 30° et 60°.

10. Installation de traitement des déchets selon la revendication 9, **caractérisée en ce que** l'angle α est de 42°.

11. Installation de traitement des déchets selon l'une quelconque des revendications 2 à 10, **caractérisée en ce qu'**un moyen de brossage (9, 10) est apte à exercer un mouvement de va-et-vient sur la grille (2).

12. Installation de traitement des déchets selon la revendication 11, **caractérisée en ce que** le moyen de brossage comprend au moins un rouleau (10) d'axe sensiblement orthogonal à la direction d'écoulement des déchets.

13. Installation de traitement des déchets selon la revendication 12, **caractérisée en ce que** le rouleau (10) est monté sur un châssis mobile (8, 9).

14. Installation de traitement des déchets selon la revendication 11, **caractérisée en ce que** le moyen de brossage comprend un bras articulé équipé d'une brosse.

## Patentansprüche

1. Verfahren zur Aufbereitung von Abfällen, insbesondere von Hausabfällen, die organische Stoffe vermischt mit unerwünschten Stoffen, insbesondere Metalle, mineralische Stoffe, Kunststoffe, Glas, enthalten, bei dem:
- die Abfälle einer ersten Sortierung durch Siebung unterzogen werden,
- der Anteil der Abfälle, die durch die Siebung durchfallen, einer Vorfermentationsbehandlung in einem Rotationsrohr mit Einspeisung an einem Ende und Extraktion an dem anderen Ende unterzogen wird,
- wobei mindestens ein Teil der Masse, die aus dem Vorfermentationsrohr austritt, einer Methanisierungsbehandlung in einem Faulbecken unterzogen wird,
- die Abfälle nach der Vorfermentationsbehandlung einer Sortierung durch Siebung unterzogen werden,
**dadurch gekennzeichnet, dass** die Sortierung durch Siebung nach der Vorfermentationsbehandlung auf einem Sieb mit einem Gitterrost (2) durchgeführt wird, der sich in einer Ebene befindet, die relativ zu der Horizontalen um einen Winkel a geneigt ist, wobei der Gitterrost (2) Rüttelbewegungen in einer Querrichtung im Wesentlichen orthogonal zu der Abführrichtung der Abfälle oberhalb des geneigten Siebes unterzogen wird.

2. Anlage für die Durchführung des Verfahrens nach Anspruch 1, umfassend:
- ein primäres Grobsieb,
- ein Vorfermentationsrotationsrohr,
- ein Faulbecken für einen Feinanteil, der aus dem Vorfermentationsrohr stammt,
- ein Grobsieb, das nach dem Vorfermentationsrotationsrohr angeordnet ist,
**dadurch gekennzeichnet, dass** das Grobsieb ein Sieb ist, das einen Gitterrost (2) umfasst, der sich in einer Ebene befindet, die relativ zu der Horizontalen um einen Winkel a geneigt ist, wobei der Gitterrost (2) Rüttelbewegungen in einer Querrichtung im Wesentlichen orthogonal zu der Abführrichtung der Abfälle oberhalb des geneigten Siebes unterzogen wird,
und dass der Gitterrost (2) eine Reihe von Drähten (5) umfasst, wobei die Mittellinie jedes Drahtes (5) eine Ausrichtung nahe der Abführrichtung der Abfälle oberhalb des Siebes aufweist, wobei sich die Drähte (5) in einer gleichen Ebene befinden, wobei der Abstand zwischen einem Draht (5) und seinen Nachbardrähten zwischen einem Maximum und einem Minimum variiert, wobei der Abstand ungleich Null ist.

3. Anlage zur Aufbereitung von Abfällen nach Anspruch 2, **dadurch gekennzeichnet, dass** die Drähte (5) im Wesentlichen sinusförmig sind.

4. Anlage zur Aufbereitung von Abfällen nach Anspruch 2 oder 3, **dadurch gekennzeichnet, dass** der Gitterrost (2) mindestens eine Querstange (6) umfasst, die senkrecht zu den Drähten (5) angeordnet und mit den Drähten (5) fest verbunden ist, um einen konstanten Abstand zwischen den Drähten (5) zu halten.

5. Anlage zur Aufbereitung von Abfällen nach Anspruch 4, **dadurch gekennzeichnet, dass** die Rüttelbewegungen auf das Sieb mittels der Querstange (6) übertragen werden.

6. Anlage zur Aufbereitung von Abfällen nach Anspruch 5, **dadurch gekennzeichnet, dass** ein elektromagnetischer Hammer (4) gegen ein Ende der Querstange (6) schlägt.

7. Anlage zur Aufbereitung von Abfällen nach einem der Ansprüche 2 bis 6, **dadurch gekennzeichnet, dass** die Masche L des Gitterrosts (2) kleiner oder gleich 5 mm ist.

8. Anlage zur Aufbereitung von Abfällen nach Anspruch 7, **dadurch gekennzeichnet, dass** die Masche L des Gitterrosts (2) kleiner oder gleich 2 mm ist.

9. Anlage zur Aufbereitung von Abfällen nach einem der Ansprüche 2 bis 8, **dadurch gekennzeichnet, dass** der Winkel a zwischen 30° und 60° beträgt.

10. Anlage zur Aufbereitung von Abfällen nach Anspruch 9, **dadurch gekennzeichnet, dass** der Winkel a 42° beträgt.

11. Anlage zur Aufbereitung von Abfällen nach einem der Ansprüche 2 bis 10, **dadurch gekennzeichnet, dass** ein Bürstenmittel (9, 10) in der Lage ist, eine Hin- und Herbewegung auf den Gitterrost (2) auszuüben.

12. Anlage zur Aufbereitung von Abfällen nach Anspruch 11, **dadurch gekennzeichnet, dass** das Bürstenmittel mindestens eine Walze (10) umfasst, deren Achse zu der Abführrichtung der Abfälle im Wesentlichen orthogonal ist.

13. Anlage zur Aufbereitung von Abfällen nach Anspruch 12, **dadurch gekennzeichnet, dass** die Walze (10) auf einem beweglichen Rahmen (8, 9) montiert ist.

14. Anlage zur Aufbereitung von Abfällen nach Anspruch 11, **dadurch gekennzeichnet, dass** das Bürstenmittel einen Gelenkarm umfasst, der mit einer Bürste versehen ist.

## Claims

1. A process for treating waste, in particular household refuse, containing organic matter mixed with undesirable products, especially metals, mineral matter, plastics, and glass, according to which process:
- the waste is subjected to a first sorting by screening,
- the fraction of waste passing through the screening is subjected to a prefermentation treatment in a rotating tube with feed at one end and extraction at the other end,
- at least one portion of the material leaving the prefermentation treatment tube is subjected to a methanization treatment in a digester,
- the waste, after the prefermentation treatment, is subjected to a sorting by screening
**characterized in that** the sorting by screening after the prefermentation treatment is performed through a sieve comprising a grate (2) located in a plane inclined by an angle *a* with respect to the horizontal, the grate (2) being subjected to vibrations in a transverse direction substantially orthogonal to the flow direction of the waste above the inclined sieve.

2. A plant for carrying out the process as claimed in claim 1, comprising:
- a first primary screen,
- a prefermentation rotating tube,
- a digester for a fine fraction resulting from the prefermentation tube,
- a screen located after the prefermentation rotating tube,
**characterized in that** the screen is a sieve comprising a grate (2) located in a plane inclined by an angle *a* with respect to the horizontal, the grate (2) being subjected to vibrations in a transverse direction substantially orthogonal to the flow direction of the waste above the inclined sieve,
and **in that** the grate (2) comprises a series of wires (5), the center line of each wire (5) having an orientation similar to the flow direction of the waste above the sieve, the wires (5) being located in one and the same plane, the distance between one wire (5) and its neighboring wires varying between a maximum and a minimum, the minimum distance being non-zero.

3. The waste treatment plant as claimed in claim 2, **characterized in that** the wires (5) have a substantially sinusoidal shape.

4. The waste treatment plant as claimed in claim 2 or 3, **characterized in that** the grate (2) comprises at least one crossbar (6), positioned perpendicularly to the wires (5) and firmly attached to the wires (5) so as to maintain a constant spacing between the wires (5).

5. The waste treatment plant as claimed in claim 4, **characterized in that** the vibrations are transmitted to the sieve by means of the crossbar (6).

6. The waste treatment plant as claimed in claim 5, **characterized in that** an electromagnetic hammer (4) hits one end of the crossbar (6).

7. The waste treatment plant as claimed in any one of claims 2 to 6, **characterized in that** the mesh L of the grate (2) is less than or equal to 5 mm.

8. The waste treatment plant as claimed in claim 7, **characterized in that** the mesh L of the grate (2) is less than or equal to 2 mm.

9. The waste treatment plant as claimed in any one of claims 2 to 8, **characterized in that** the angle *a* is between 30° and 60°.

10. The waste treatment plant as claimed in claim 9, **characterized in that** the angle *a* is 42°.

11. The waste treatment plant as claimed in any one of claims 2 to 10, **characterized in that** a brushing means (9, 10) is capable of exerting a back-and-forth movement over the grate (2).

12. The waste treatment plant as claimed in claim 11, **characterized in that** the brushing means comprises at least one roller (10) having an axis substantially orthogonal to the flow direction of the waste.

13. The waste treatment plant as claimed in claim 12, **characterized in that** the roller (10) is mounted on a movable frame (8, 9).

14. The waste treatment plant as claimed in claim 11, **characterized in that** the brushing means comprises an articulated arm equipped with a brush.
